Europäisches Patentamt

European Patent Office

Office européen des brevets

(19)

(11) Publication number: **0 190 972**
A2

(12) **EUROPEAN PATENT APPLICATION**

(21) Application number: 86400216.7

(22) Date of filing: 31.01.86

(51) Int. Cl.⁴: **C 12 P 21/00**
//A61K39/29

(30) Priority: 01.02.85 JP 18201/85

(43) Date of publication of application:
13.08.86 Bulletin 86/33

(84) Designated Contracting States:
AT BE CH DE FR GB IT LI LU NL SE

(71) Applicant: MITSUBISHI CHEMICAL INDUSTRIES
LIMITED
5-2, Marunouchi 2-chome Chiyoda-ku
Tokyo 100(JP)

(71) Applicant: Ono, Yasushi
1-7-23 Niikura
Wako-shi Saitama-ken(JP)

(71) Applicant: Shikata Toshio
1-172 Motokitakata
Ichikawa-shi Chiba-ken(JP)

(71) Applicant: Shimizu Yohko
3-17-23 Nishiwaseda Shinjuku-ku
Tokyo(JP)

(72) Inventor: Ono, Yasushi
1-7-23 Niikura
Wako-shi Saitama-ken(JP)

(72) Inventor: Shikata, Toshio
1-172 Motokitakata
Ichikawa-shi Chiba-ken(JP)

(72) Inventor: Shimizu, Yohko
3-17-23 Nishiwaseda Shinjuku-ku
Tokyo(JP)

(72) Inventor: Yamada, Ei
442-26 Hirao
Inagi-shi Tokyo(JP)

(72) Inventor: Honda, Yoshikazu
2-3-16 Nishimikado
Kamakura-shi Kanagawa-ken(JP)

(74) Representative: Gutmann, Ernest et al,
S.C. Ernest Gutmann - Yves Plasseraud 67, boulevard
Haussmann
F-75008 Paris(FR)

(54) Non-A non-B hepatitis antigens.

(57) Disclosed herein is an antigen specific to non-A non-B
hepatitis. The antigen has a density of approximately 1.17 to
1.26 by sucrose density gradient centrifugation. The antigen
reacts with an antibody which is obtained by transforming
lymphocytes from a human or chimpanzee individual suffer-
ing from non-A non-B hepatitis with EB virus, screening the
transformed cells for the non-A non-B hepatitis related
antibody, and cloning positive cells.

EP 0 190 972 A2

## TITLE OF THE INVENTION

### NON-A NON-B HEPATITIS ANTIGENS

## BACKGROUND OF THE INVENTION

### Field of the Invention:

This invention relates to an antigen directed to non-A non-B hepatitis.

### Description of the Prior Art:

Among viral hepatitis, pathogenic viruses of hepatitis type A and type B have been found and, therefore, an immunologic diagnosis of the diseases has now been possible.

However, a pathogenic virus of another type of hepatitis different from type A and type B, which is called non-A non-B type and said to account for over 90% of post-transfusion hepatitis, has not yet been identified. One fact which has already been established is that a chimpanzee may be infected with human non-A non-B hepatitis. Many studies for searching an antigen-antibody system related to the non-A non-B hepatitis have been done using mainly sera from patients; nevertheless, no effective system has been found.

In order to find such an antigen-antibody system related to non-A non-B hepatitis, we have done various investigations and found an antigen specific to the non-A non-B hepatitis.

## SUMMARY OF THE INVENTION

Accordingly, the principal object of this invention is to provide an antigen directed to non-A non-B hepatitis.

Thus, this invention comprises an antigen specific to non-A non-B hepatitis, having a density of approximately 1.17 to 1.26 by sucrose density gradient centrifugation, and reacting with an antibody which is obtained by transforming lymphocytes of an individual suffering from non-A non-B hepatitis with EB virus, screening the transformed cells for the non-A non-B hepatitis-related antibody, and cloning the resulting positive cells.

## DESCRIPTION OF THE INVENTION

This invention will be fully described hereinafter.

This invention provides an antigen specific for non-A non-B hepatitis.

The density of the antigen is approximately 1.17 to 1.26 as determined by sucrose density gradient centrifugation.

The antigen according to this invention will react with an antibody related to non-A non-B hepatitis, said antibody being obtained by transforming lymphocytes of an individual suffering from non-A non-B hepatitis with EB virus, screening the transformed cells for the non-A non-B hepatitis-related antibody, and cloning the resulting positive cells. The method for obtaining such an antibody will be more fully described later.

The antigen according to this invention may be prepared by any conventional method; one example of such methods will be illustrated below:

Liver specimens derived from a human or chimpanzee individual with non-A non-B hepatitis may generally be utilized for an antigenic material. Thus, a liver specimen is homogenized, centrifuged at about 8,000 to 10,000 r.p.m. for about 30 minutes to one hour, and ultracentrifuged at about 100,000 g. The precipitate may optionally be subjected to ion exchange chromatography or affinity column chromatography prior to the subsequent density gradient centrifugation described below. The resultant precipitate can be further subjected to gel filtration chromatography to eliminate ferritin contained therein at any step in the preparation course.

The precipitate obtained from the ultracentrifugation may further be purified by density gradient centrifugation with sucrose, CsCl, KBr, or the like. This purification is carried out while assaying the antigen for the reactivity with an antibody related to non-A non-B hepatitis.

Such a non-A non-B hepatitis-related antibody which may be used in the assay is obtained by a method wherein lymphocytes from an individual with non-A non-B hepatitis, preferably at the stage of recovery, are transformed with Epstein-Barr (EB) virus to obtain a culture of cells positive to the non-A non-B hepatitis-related antibody, and the culture is then cloned. Preferably, the antibody has the ability to afford defense against infection of non-A non-B hepatitis.

The method of preparing such an antibody used in the purification of the antigen according to this invention will be illustrated hereinbelow by way of example.

Peripheral blood is exsanguinated from a human or chimpanzee at the stage of recovery from non-A non-B hepatitis and heparinized. Lymphocytes are separated by Ficol-Conray density centrifugation.

On the other hand, cells producing and releasing EB virus, e.g. B95-8, are cultivated in a medium. The supernatant is separated and used as a viral source, i.e. EB viral solution.

After contacting the lymphocytes with the EB viral solution, the cells are inoculated in a microtiter plate for tissue culture at different cell concentrations and cultivated.

The transformation of lymphocytes with EB virus may be performed by any appropriate conventional method; the oligo clone method in which cells are inoculated at low cell densities is preferably utilized.

During the period when the transformed cells are growing, the supernatant is collected and screened for the antibody activity using liver specimen with non-A non-B hepatitis. Cells positive in the antibody activity are then cloned.

Cloning may be performed by any conventional method, such as soft agar culture, limiting dilution, and single cell manipulation method; soft agar or limiting dilution method is usually used.

In the soft agar method, cells are inoculated in

- 4 -

a density in a soft agar layer on an anchor agar layer, growing colonies are separated and again transferred to the suspension culture, and antibody positive clones are collected.

In the limiting dilution method, by using B cells tumorized by X ray irradiation as feeder cells, cells are inoculated in a density in a microtiter plate for tissue culture to obtain antibody positive clones.

The antibodies produced by the cloned cell strains can be collected by any conventional method. These antibodies react specifically with antigens appearing in liver cells with non-A non-B hepatitis.

The obtained antibody can be purified by any conventional method, for example, gel filtration chromatography with "Sephacryl S-300", etc.

The antibody is a human or chimpanzee IgM antibody having a molecular weight of approximately 900,000 by sucrose density gradient centrifugation and gel filtration and comprising H chain with a molecular weight by SDS-polyacrylamide gel electrophoresis of approximately 63,000 and L chain of approximately 23,000.

Generally, the assay performed in the purification of the antigen according to this invention is carried out by using the antibody obtained above in radioimmunoassay (RIA). Enzyme-linked immunosorbent assay (ELISA), passive hemagglutination (PHA) and other assay methods can also be utilized herein.

The thus obtained antigen according to this invention has a density by sucrose density gradient centrifugation of approximately 1.17 to 1.26, reacts with the above mentioned antibody, and is thus specific to non-A non-B hepatitis.

The antigen according to this invention may be utilized in many applications.

For example, sera from patients with non-A non-B hepatitis can be tested for the antibody activity directed to the antigen of this invention, enabling the diagnosis of the case history for non-A non-B hepatitis. Further, immunization of an animal, such as rabbit and mouse, with the antigen may provide a stable polyclonal or monoclonal antibody.

In addition, if the antigenic determinant could be putatively determined by analyzing the primary or higher order structure of the antigen according to this invention and an analogous peptide exhibiting the antigenic property of the determinant could be synthesized, antigens useful for diagnosis or vaccine materials, which can be up to today supplied only in a small amount from natural sources, could be produced artificially in a large amount.

Moreover, when the analysis of the primary structure, i.e. amino acid sequence, of the antigen according to this invention will be completed, cDNA corresponding to the antigen could be prepared and such antigens would be produced by recombinant DNA technique.

## DESCRIPTION OF THE PREFERRED EMBODIMENTS

The invention will be more fully illustrated by the following non-limiting examples.

Exmaple 1:  Isolation and Purification of Antigens

(I)  Isolation and purification of antigens appearing in chimpanzee liver cells with non-A non-B hepatitis:

(1)  A hepatocyte mass, 35 g, of a chimpanzee suffering from non-A non-B hepatitis was minced into small pieces and suspended in cold Tris buffer (50 mM Tris-HCl buffer containing 0.15 M NaCl and 1 mM EDTA, pH 7.5) so that the total volume was 350 ml.  The hepatocyte suspension was homogenized in a ultra high universal homogenizer, HISCOTRON, K.K. NICHION IRIKA KIKAI SEISAKUJO, Japan, the vessel wall of which was cooled at 0°C, and centrifuged at 8,000 rpm, 4°C for one hour.  Thus, 300 ml of supernatant was obtained.

(2)  Samples of the supernatant were ultracentrifuged at 30,000 rpm, 4°C for 20 hours in RP-42 rotor, Hitachi. The separated supernatant and the precipitate which was suspended and solubilized in 50 ml of cold Tris buffer were each tested for the antigen activity by the radioimmunoassay described hereinbelow.  The results showed that 95% or more antigen was contained in the precipitate.

(3)     The obtained antigen fraction (17 ml) was layered on 2 ml of 60% sucrose and 20 ml of 30% sucrose (w/v, in Tris buffer) in a centrifugal tube in triplicate, and ultracentrifuged at 25,000 rpm, 4°C for 24 hours in RPS-25 rotor,

Hitachi.  Antigen activities were measured by RIA in the supernatant from the upper layer of 60% sucrose and in the precipitate which was suspended and solubilized in 10 ml of cold Tris buffer.  The precipitate contained 95% or more antigen.

(4)     The thus obtained antigen fraction in two tubes (5 ml) was subjected to non-equilibrium gradient centrifugation with 10-45% (w/w)  sucrose density gradient Tris buffer in RPS-25 rotor, Hitachi, at 25,000 rpm, 4°C for one hour to fractionize the fraction into one ml per tube.

Each of the obtained fractions was tested for the antigen activity by RIA.  The antigen was collected in fractions of 18-41% sucrose.

(5)     The resultant antigen fractions (fraction I:

18-25% sucrose; fraction II:   25-33%; fraction III:   33-41%) were each concentrated to 0.5 ml and subjected to ultracentrifugation with 12 ml of 10-60% (w/w) sucrose density gradient Tris buffer in RPS-40T rotor, Hitachi, at 24,000 rpm, 4°C for 72 hours to fractionize into 0.7 ml per tube.

Each fraction was assayed for the antigen activity by RIA.

The antigen was collected in fractions with sucrose density of 1.17 to 1.26 from all fractions I, II and III.

(II)    Isolation and purification of antigens appearing in

chimpanzee liver cells with non-A non-B hepatitis:

(1)        A hepatocyte mass, 36 g, of a chimpanzee suffering from non-A non-B hepatitis was minced into small pieces and suspended in cold Tris buffer (50 mM Tris-HCl buffer

containing 0.15 M NaCl and 1 mM EDTA, pH 7.5) so that the total volume was 350 ml.  The hepatocyte suspension was homogenized in a ultra high universal homogenizer, HISCOTRON, K.K. NICHION IRIKA KIKAI SEISAKUJO, Japan, the vessel wall of which was cooled at 0°C, and centrifuged at 8,000 rpm, 4°C for one hour.  Thus, 300 ml of supernatant was obtained.

(2)        The resultant concentrated sample (22 ml) was layered on 3 ml of 60% sucrose and 20 ml of 20% sucrose (w/v in Tris buffer) in a centrifugal tube in duplicate, and ultracentrifuged at 30,000 rpm, 4°C for 2 hours in RP-42 rotor, Hitachi.  Antigen activities were measured by RIA in the supernatant from the upper layer of 60% sucrose and in the precipitate which was suspended and solubilized in 14 ml of cold Tris buffer.  The precipitate contained 95% or more antigen.

(3)        The thus obtained antigen fraction in two tubes (7 ml) was subjected to ultracentrifugation (10-60%, w/w,

sucrose density gradient, Tris buffer, RPS-25 rotor, Hitachi, 24,000 rpm, 4°C, 20 hours) to fractionize into 1.7 ml per tube.  Each of the resulting fractions was tested for the antigen activity by RIA.  The antigen was collected in fractions with sucrose density of 1.17 to 1.26.

(4)     The sucrose density gradient centrifugation described in (I) (5) was repeated to collect the antigen in fractions having sucrose density of 1.17 to 1.26.

(5)     The thus obtained antigen fraction was subjected to "Sepharose" 4B column (1 x 50 cm) chromatography in Tris buffer to eliminate ferritin contained in the antigen fraction.

(III)   Radioimmunoassay (RIA):

(i)     Iodine-labelling of antibodies

1.    One ml of 0.2 M sodium borate buffer, pH 9.0, containing 10% bovine serum albumin (BSA) is applied to "Sephadex" G-25 column, 0.7 x 20 cm, to block the resin with albumin.

2.    $Na^{125}I$ (500 μCi) is distributed into polystyrene tubes, each in 75 x 12 mm.

3.    Fifty μl IgM antibody, which is obtained in Reference Example 1 described hereinbelow (50 μg in 0.2 M sodium borate buffer, pH 9.0), is added to the tube and well stirred.

4.    Fifty μl chloramine T solution (1 mg/ml in 0.2 M sodium borate buffer, pH 9.0) is added and well stirred to react at room temperature for one minute.

5.    Fifty μl sodium metabisulfite (2.5 mg/ml in 0.2 M sodium borate buffer, pH 9.0) is added and well stirred.

6.    Three hundred μl potassium iodide solution (1 mg/ml in sodium borate buffer, pH 9.0, containing 0.1% BSA) is added and well stirred.

7.  IgM fraction is fractionized on "Sephadex" G-25 column.

(ii)  Radioimmunoassay

1.  IgM antibody (100 µg/ml) solubilized in 0.2 M sodium borate buffer, pH 9.0, is adsorbed on "Falcon 3912" 96-well microtiter plate at 4°C over a whole day and night.

2.  The antibody solution is removed, and the plate is washed with 0.01 M phosphate buffer solution (PBS), pH 7.2, to block the plate with 5% BSA-PBS.

3.  A standard antigen or test antigen fraction is diluted with 0.25% gelatin-PBS containing 0.05% NP-40 and 0.02% $NaN_3$, and 45 µl of the diluted antigen solution is distributed in duplicate to react at room temperature for 2 to 16 hours.

4.  The plate is washed with PBS-"Tween 20" (0.1%).

5.  $^{125}I$-labelled antibody is diluted with gelatin-PBS, and 50 µl solution ($5 \times 10^4$ cpm) is added to each well and reacted at room temperature for 2 to 16 hours.

6.  The plate is washed with PBS-"Tween 20".

7.  Each well is cut and radioactivity is measured by a gamma counter.

Reference Example 1:  Preparation of Antibodies against Non-A Non-B Hepatitis

1.  Transformation of human peripheral blood lymphocytes at the stage of recovery from non-A non-B hepatitis:

(1) Culture Medium

To RPMI 1640 medium, Idenshi Jikkenho Koza II, "Experiments for cell culture genes", Kyoritsu Shuppan KK, Japan (1981), 100 µg/ml lilacillin, 100 µg/ml streptomycin, 2 mM glutamine and 1.6 g/l sodium bicarbonate were added. Carbon dioxide was then blown into the medium to adjust the pH to 7.0-7.4, and fetal calf serum (FCS) was added to 20%.

(2) Preparation of EB Viral Solution

B95-8 cells producing EB virus, Proceedings of National Academy of Science, 70(1), 190-194 (1973), were cultivated at cell density of 3 x $10^5$/ml of RPMI 1640 + 10% FCS medium for 7 days and filtrated by a Millipore filter of 0.45 µm. The resultant supernatant was used as a viral solution. Viral titer is expressed by $TD_{50}$/ml evaluated by using transformation of umbilical cord blood. Viral solutions with their titer of $10^5$ $TD_{50}$/ml or more were used.

(3) Separation of Lymphocytes

Human venous blood derived from a patient with non-A non-B hepatitis at the stage of recovery with the addition of heparin was subjected to density centrifugation with Ficol-Conray of 1.077 to separate monocytes which was used as peripheral blood lymphocytes. In this procedure, about $10^6$ peripheral blood lymphocytes were separated per ml of the peripheral blood.

(4) Adsorption of EB Virus and Culture

EB viral solution having $TD_{50}$ of $10^5$/ml or more

was added to the peripheral blood lymphocytes precipitated by centrifugation in an amount of 1 ml of solution per $10^6$

lymphocytes, and allowed to stand at 37°C for one hour.

After removing the viral solution by centrifugation, RPMI 1640 + 20% FCS medium was added to adjust the cell density and 0.1 ml of the cells were inoculated to each well of a flat bottom microtiter plate.  On the fourth day 0.1 ml of the medium was added to each well and thereafter a half of the medium was changed with fresh medium  once per 4 to 7 days.  When $10^5$ cells were inoculated into each well of the flat bottom microtiter plate cell colonies were formed and well proliferated by 7 to 10 days, although this condition will vary depending on the number of cells inoculated.

Generally, $10^3$ to $10^5$ of peripheral blood lymphocytes to which EB virus has been adsorbed are inoculated to each well of a flat bottom microtiter plate.

(5)   Detection of Non-A Non-B Hepatitis-Related Antibodies in Supernatant

In the third or fourth week from the transformation with EB virus, the supernatant was collected and assayed for the antibody titer when the cells had grown to observe wells positive in the antibody activity, although the period of time for the culture until such detection will vary with the number of cells inoculated.

The antibody-positive wells were then transferred to 24 multiwell plate, 35 mm petri dish, and then 60 mm petri dish for further culture.  Cloning was started in the sixth week from the transformation of the cells.

2.  Cloning:

Cloning was performed by soft agar culture.

RPMI 1640 + 20% FCS medium containing agarose, from Marine Colloids, "Sea Plaque" agarose, which comprised 0.5% of a supporting agar layer and 0.3% of a soft agar inoculating layer, was used.  100 to 10,000 cells were inoculated in each plate. 10 to 20 days after, colonies which could be seen by naked eyes were formed.  The colonies were removed by a Pasteur pipet and transferred to a microtiter plate in which 0.1 ml of RPMI 1640 + 20% FCS had previously been added.  The cells were cultured to provide clone strains.

The cloned cells, designated as S-1 cells, have been deposited with "Collection nationale de cultures de micro-organismes" (C.N.C.M.), Institut Pasteur, Paris, on January 22, 1986 under accession number C.N.C.M. I-510.

3.  Collection and Purification of Antibodies:

The clone was propagated in serum free medium containing 0.5 % bovine serum albumin.  The supernatant was collected to obtain antibodies.  The supernatant was ultrafiltrated to exclude molecules having molecular weights of 300,000 or more, and purified by gel filtration chromatography on "Sephacryl S-300" using 0.2 M borate buffer, pH 9.0.  The resulting purified human antibody represented a precipitation curve corresponding to IgM antibody in Ouchterlony's diffusion method and immunoelectrophoresis.  Analysis by SDS electrophoresis under reductive condition showed two bands corresponding to H and L chains of IgM.

Reference Example 2:    Reactivities of the Antibody with Liver

Specimens

(1)    Reactivities with liver specimens of non-A non-B hepatitis:

The antibody obtained in Reference Example 1 was assayed for the presence of the reactivity with liver specimens with non-A non-B hepatitis by immunofluorescence method.

Thus, liver specimens sliced by a cryostat were treated with acetone at room temperature for 10 minutes. After drying, the non-A non-B hepatitis-related antibody as a primary antibody was placed on the slice and allowed to react at 37°C for 60 minutes in a wet box.  The antibody solution had previously been diluted to an appropriate concentration.  After washing out the primary antibody with PBS, the specimen was washed in PBS with stirring. Then, FITC-labelled anti-human IgM antibody as a secondary antibody was reacted with the specimen at 37°C for 60 minutes in a similar manner as described above.  After washing out the  labelled antibody with PBS, the specimen was washed in PBS with stirring and air dried.  A sealing agent, glycerin: 0.5 M $Na_2CO_3$-$NaHCO_3$ buffer (9:1), was dripped and a cover glass was mounted thereon.  The specimen was observed by a fluorescence microscope with a mercury lamp as a light source.

The results showed that the antibody reacted with the antigen appearing in the liver with non-A non-B hepatitis.

- 15 -

(2) Reactivities with liver specimens of other hepatitis and normal liver specimen:

In the same manner as described in (1) above, the antibody obtained in Reference Example 1 was assayed for the reactivities with liver specimens other than those of non-A non-B hepatitis by immunofluorescence method.

The results showed that the antibody did not react with liver specimens of hepatitis type A or B, nor with those of non-viral hepatitis, nor with normal liver specimens.

Reference Example 3:  Estimation of Infection Protecting

Abilities

(1)      Infected sera were prepared from blood of a chimpanzee suffering from non-A non-B hepatitis.

Two ml of the infected sera were reacted with 0.5 ml of the human IgM monoclonal antibody, 1 OD (280 nm)/ml, obtained in Reference Example 1, at 37°C for one hour and allowed to stand at 4°C overnight. Anti-human IgM antibody, Hoechst, was then added, reacted at 37°C for 2 hours and allowed to stand at 4°C overnight. The sample was then centrifuged at 10,000 rpm for 10 minutes. The obtained supernatant was intravenously injected to a normal chimpanzee in an amount of 2 ml. After 9 weeks non-A non-B hepatitis did not appear.

(2)      On the other hand, the same procedure was repeated except that the human monoclonal antibody was replaced by anti-SRBC antibody, antibody against sheep red cell surface antigen (1 OD 280 nm/ml). Non-A non-B hepatitis appeared in a chimpanzee after 2 or 3 weeks.

WHAT IS CLAIMED IS:

    1.  An antigen specific to non-A non-B hepatitis, which has a density by sucrose density gradient centrifugation of approximately 1.17 to 1.26, and which reacts with an antibody obtained by transforming lymphocytes of an individual suffering from non-A non-B hepatitis with EB virus, screening the transformed cells for the non-A non-B hepatitis related antibody, and cloning positive cells.